# FASCICULE DE BREVET EUROPEEN

(11) **EP 1 723 431 B1**
(45) Date de publication et mention de la délivrance du brevet: **05.09.2012**
(21) Numéro de dépôt: 05733006.0
(22) Date de dépôt: 02.03.2005
(51) Int. Cl.: G01N 33/543, G01N 33/58, G01N 33/533, G01N 33/52, G01N 33/553, A61K 41/00, A61K 49/00, A61K 49/18, C01F 17/00, B82Y 30/00, B82Y 5/00, C08L 83/04, A61K 47/00

(54) **NANOPARTICULES HYBRIDES COMPRENANT UN COEUR DE LN2O3 PORTEUSES DE LIGANDS BIOLOGIQUES ET LEUR PROCEDE DE PREPARATION**
HYBRIDNANOPARTIKEL MIT EINEM LN2O3-KERN UND MIT BIOLIGANDEN SOWIE VERFAHREN ZUR HERSTELLUNG DAVON
HYBRID NANOPARTICLES INCLUDING AN LN2O3 CORE AND HAVING BIOLIGANDS, AND METHOD FOR PREPARING SAME

(30) Priorité: 02.03.2004 FR 0402115
(43) Date de publication de la demande: 22.11.2006
(73) Titulaire: Université Claude Bernard Lyon I, 69622 Villeurbanne Cedex (FR); CENTRE NATIONAL DE LA RECHERCHE SCIENTIFIQUE (CNRS), 75794 Paris Cedex 16 (FR); Institut National des Sciences Appliquées de Lyon, 69621 Villeurbanne (FR)
(72) Inventeur: PERRIAT, Pascal, F-69005 Lyon (FR); LOUIS, Cédric, F-38138 Les Côtes d'Arey (FR); MARQUETTE, Christophe, F-69006 Lyon (FR); BAZZI, Rana, F-69100 Villeurbanne (FR); ROUX, Stéphane, F-69100 Villeurbanne (FR); TILLEMENT, Olivier, F-69270 Fontaine Saint Martin (FR); LEDOUX, Gilles, F-69006 Lyon (FR)
(74) Mandataire: Sarlin, Laure V.
(86) Numéro de dépôt international: PCT/FR2005/000491
(87) Numéro de publication internationale: WO 2005/088314

(56) Documents cités:
- US-A1- 2003 180 780
- US-B1- 6 368 586
- FENG J ET AL: "FUNCTIONALIZED EUROPIUM OXIDE NANOPARTICLES USED AS A FLUORESCENT LABEL IN AN IMMUNOASSAY FOR ATRAZINE" ANALYTICAL CHEMISTRY, AMERICAN CHEMICAL SOCIETY. COLUMBUS, US, vol. 75, no. 19, 1 octobre 2003 (2003-10-01), pages 5282-5286, XP001184591 ISSN: 0003-2700
- BAZZI R ET AL: "Synthesis and luminescent properties of sub-5-nm lanthanide oxides nanoparticles" JOURNAL OF LUMINESCENCE, AMSTERDAM, NL, vol. 102-103, mai 2003 (2003-05), pages 445-450, XP004416620 ISSN: 0022-2313
- CANNAS, C. ET AL: "Synthesis, characterization and optical spectroscopy of a Y2O3-SiO2 nanocomposite doped with Eu3+" JOURNAL OF NON-CRYSTALLINE SOLIDS, CODEN: JNCSBJ; ISSN: 0022-3093, vol. 306, no. 2, août 2002 (2002-08), pages 193-199, XP004361999
- QUE, WENXIU ET AL: "Yellow-to-violet upconversion in neodymium oxide nanocrystal /titania/ormosil composite sol-gel thin films derived at low temperature" JOURNAL OF APPLIED PHYSICS, CODEN: JAPIAU; ISSN: 0021-8979, vol. 90, no. 9, 1 novembre 2001 (2001-11-01), pages 4865-4867, XP012054452
- QUE, W. ET AL: "Fluorescence characteristics from microemulsion technique derived erbium (III) oxide nanocrystals" MATERIALS RESEARCH BULLETIN, CODEN: MRBUAC; ISSN: 0025-5408, vol. 36, no. 5-6, mars 2001 (2001-03), - avril 2001 (2001-04) pages 889-895, XP004239552
- DATABASE CHEMABS [Online] CHEMICAL ABSTRACTS SERVICE, COLUMBUS, OHIO, US; 8 mars 2004 (2004-03-08), SUN, BAOQUAN ET AL: "Rare earth nanoparticle for labeling biomaterial, its preparation and application" XP002300295 extrait de STN Database accession no. 2004:184856 & CN 1 378 083 CN (QINGHUA UNIVERSITY, PEOP. REP. CHINA) 30 mars 2001 (2001-03-30)
- LOUIS, CEDRIC ET AL: "Nanosized hybrid particles with double luminescence for biological labeling" CHEMISTRY OF MATERIALS, CODEN: CMATEX; ISSN: 0897-4756, vol. 17, no. 7, 5 avril 2005 (2005-04-05), pages 1673-1682, XP002333998
- BRIDOT J-L ET AL: "Hybrid gadolinium oxide nanoparticles: Multimodal contrast agents for in vivo imaging", JOURNAL OF THE AMERICAN CHEMICAL SOCIETY, AMERICAN CHEMICAL SOCIETY, WASHINGTON, DC; US, vol. 129, no. 16, 31 March 2007 (2007-03-31), pages 5076-5084, XP002481277, ISSN: 0002-7863, DOI: 10.1021/JA068356J [retrieved on 2007-03-31]
- BRIDOT JEAN-LUC ET AL: "Hybrid gadolinium oxide nanoparticles combining imaging and therapy", JOURNAL OF MATERIALS CHEMISTRY, THE ROYAL SOCIETY OF CHEMISTRY, CAMBRIDGE, GB, vol. 19, no. 16, 1 January 2009 (2009-01-01), pages 2328-2335, XP002615956, ISSN: 0959-9428, DOI: 10.1039/B815836C [retrieved on 2009-03-02]
- LEWIN M ET AL: "Tat peptide-derivatized magnetic nanoparticles allow in vivo tracking and recovery of progenitor cells", NATURE BIOTECHNOLOGY, NATURE PUBLISHING GROUP, NEW YORK, NY, US, vol. 18, 1 April 2000 (2000-04-01), pages 410-414, XP002958421, ISSN: 1087-0156, DOI: 10.1038/74464
- BAZZI R ET AL: "Synthesis and properties of europium-based phosphors on the nanometer scale: Eu2O3, Gd2O3:Eu, and Y2O3:Eu", JOURNAL OF COLLOID AND INTERFACE SCIENCE, ACADEMIC PRESS, NEW YORK, NY, US, vol. 273, no. 1, 1 May 2004 (2004-05-01), pages 191-197, XP027132340, ISSN: 0021-9797 [retrieved on 2004-04-02]
- PRIETO-SIMON B ET AL: "Metal-Dispersed Xerogel-Based Composite Films for the Development of Interference Free Oxidase-Based Biosensors", CHEMISTRY OF MATERIALS 20040323 AMERICAN CHEMICAL SOCIETY US, vol. 16, no. 6, 27 February 2004 (2004-02-27), pages 1026-1034, XP055008418, DOI: DOI:10.1021/CM035110U

## Description

La présente invention concerne le domaine technique des sondes pour la détection, le suivi et la quantification dans les systèmes biologiques. Plus particulièrement, l'invention a pour objet de nouvelles particules sondes hybrides dont le coeur est constitué par une nanoparticule à base de sesquioxyde de lanthanide, de diamètre moyen inférieur à 10 nm, sur laquelle sont immobilisées des molécules sondes, ainsi que leur procédé de préparation.

L'emploi de sondes associées à un marqueur, dans les systèmes biologiques pour la détection (reconnaissance) ou le suivi de substances spécifiques, appelées cibles, est une technique usuelle dans le domaine du diagnostic médical et de la recherche en biologie. De telles sondes sont particulièrement utilisées pour la cytométrie de flux, l'histologie, les tests immunologiques ou la microscopie de fluorescence, aussi bien pour l'étude de matériaux biologiques que de matériaux non biologiques.

Des systèmes de marquage usuels sont, par exemple, des isotopes radioactifs de l'iode, du phosphore et d'autres éléments comme l'enzyme peroxydase ou la phosphatase alcaline dont la détection nécessite un substrat particulier. Dans la plupart des cas, le couplage sélectif entre le marqueur et la substance à détecter est effectué par une seule ou une association de molécules fonctionnelles. La sélectivité de la liaison est essentielle afin d'identifier sans ambiguïté la substance cible à détecter. Les réactions assurant le couplage sont connues et décrites par exemple dans « Bioconjugate Techniques », G. T. Hermanson, Academic Press, 1996 ou dans « Fluorescent and Luminescent Probes for Biological Activity. A Practical Guide to Technology for Quantitative Real-Time Analysis », Second Edition, W. T. Mason, ed., Academic Press, 1999.

Les colorants organiques fluorescents sont très utilisés pour le marquage. Il s'agit de la fluorescéine, du Texas Red ou de Cy5, qui sont sélectivement liés à une substance biologique ou organique déterminée jouant le rôle de sonde. Après excitation de la sonde marquée par une source externe, le plus souvent électromagnétique, la présence des substances biologiques ou organiques cibles liées à la sonde est mise en évidence par l'émission de fluorescence de la part de cette dernière.

Un des principaux inconvénients des colorants organiques fluorescents, à la base des techniques actuelles, réside dans leur faible stabilité chimique : ils sont dégradés, voire décomposés après quelques millions de cycles d'absorption et d'émission de lumière en présence d'acides ou de radicaux. Ils montrent également, pour la plupart des applications, une stabilité insuffisante vis-à-vis de la lumière incidente. De plus, les colorants organiques fluorescents peuvent exercer des effets phototoxiques pour l'environnement biologique.

Les colorants organiques fluorescents présentent également l'inconvénient d'émettre sur une large gamme du spectre et d'être excités par un rayonnement dont la longueur d'onde se situe dans une fenêtre très étroite. A cause de cela, l'identification simultanée de plusieurs substances marquées chacune par des colorants fluorescents différents, appelée également multiplexing, est rendue difficile par le recouvrement des bandes d'émission, et le comptage des substances marquées différemment est limité. D'autre part, l'excitation efficace des différents colorants nécessite plusieurs sources de lumière, en général des lasers, ou l'utilisation d'un montage optique complexe comprenant une série de filtres lorsque la lumière blanche est utilisée comme source excitatrice.

L'art antérieur propose déjà des alternatives à l'utilisation de colorants organiques fluorescents. Comme première alternative, il est possible d'utiliser des complexes métalliques (ligands chélatants) avec un ion métallique de la classe des lanthanides comme marqueur fluorescent (brevets US 4,637,988 et US 5,891,656). Le principal avantage de ces systèmes est que les objets excités présentent une longue durée de vie qui peut atteindre quelques millisecondes ce qui permet d'envisager des expériences avec résolution temporelle. Cependant l'utilisation de ces chélates de lanthanide est gênée par la très forte dilution de la luminescence (quenching) en milieu aqueux. Cela restreint considérablement le domaine d'application des chélates car les milieux biologiques sont généralement très riches en eau. Des tentatives de séparer la substance à détecter du milieu biologique pour la placer dans un environnement anhydre ont donc été réalisées (I. Hemmilä, Scand. J. Clin. Lab. Invest. 48,1988, pages 389-400) ; elles ne rendent cependant pas possibles les examens immunohistochimiques car l'information concernant l'endroit du marquage est perdue au cours de l'étape de séparation. Dans les brevets US 4,283,382 et US 4,259,313, il est indiqué que des particules de polymère (latex) dans lesquelles des chélates de lanthanides sont emprisonnés peuvent être employées comme marqueurs fluorescents.

Maïté Lewin et al. dans Nature biotechnology, vol. 18, April 2000, 410-414 décrit des nanoparticules fonctionnalisées avec des Tat-peptides. Les nanoparticules consistent en un petit monocrystal (5nm) composé d'un coeur en oxyde de fer superparamagnetic stabilisé avec un enrobage en dextran aminé réticulé. De plus l'enrobage de dextran contient un radiotraceur (¹¹¹In).

Une autre alternative proposée par l'art antérieur consiste à marquer la sonde destinée à se lier avec la cible à détecter, avec des particules intrinsèquement luminescentes. En particulier, des nanoparticules de matériau semi-conducteur ont donné lieu à d'intenses recherches. Le brevet US 5,990,479, et les demandes de brevet internationales publiées sous le numéro WO 00/17642 et WO 00/29617 montrent que les nanocristaux semiconducteurs fluorescents, qui appartiennent à la classe des éléments II-VI ou III-V et ceux qui , sous certaines conditions, sont composés des éléments du 4^{ème} groupe principal du tableau périodique, peuvent être utilisés comme marqueur fluorescent pour les systèmes biologiques. En raison du phénomène connu sous le vocable « quantum size effect », la longueur d'onde d'émission d'un nanocristal semiconducteur fluorescent est imposée par sa taille. Ainsi, en faisant varier la taille de ces nanocristaux, une large gamme du spectre peut être couverte, de la lumière visible au proche infra-rouge. Leur utilisation comme marqueur biologique est décrite par Warren C.W. Chan, Shuming Nie, Science, 281, 2016-2018, 1998, et par Marcel Bruchez Jr, Mario Moronne, Peter Gin, Shimon Weiss, A. Paul Alivisatos, Science, 281, 2013-2016, 1998. La préparation de nanocristaux semiconducteurs avec une longueur d'onde d'émission bien définie, c'est-à-dire avec une faible dispersion en taille, exige une très grande précision et nécessite une parfaite maîtrise des conditions opératoires et du déroulement de la synthèse. Ils sont, par conséquent, très difficiles à produire. La palette étendue de couleurs qu'offrent ces cristaux semiconducteurs résulte d'une variation de taille de l'ordre de quelques Angström (c'est-à-dire quelques couches atomiques). Les synthèses en solution permettent rarement d'atteindre un tel degré de précision. De plus, la recombinaison de paires électron-trou observée à la surface des nanocristaux limite le rendement quantique à une faible valeur.

Pour contourner ce problème, une structure coeur / coquille (« core / shell ») a été proposée : il s'agit d'enrober individuellement les nanocristaux semiconducteurs fluorescents par une couche de matériau semi-conducteur avec un plus large gap (ZnS, CdS). De plus, le marquage sélectif de biomolécules par des nanocristaux semi-conducteurs fluorescents nécessite la formation d'une couche de polysiloxane fonctionnalisé par des groupes amines (époxy et acide carboxylique). Ces derniers constitueront des points d'ancrage pour les biomolécules. La préparation de ces nanocristaux demande, donc, au moins trois étapes de synthèse dont les deux premières sont très délicates et est donc difficilement industrialisable.

Le marquage par des nanoparticules d'oxyde rendues luminescentes grâce au dopage par des ions luminescents (terre rare) n'est pas, malgré des résultats prometteurs, encore très répandu. Son principal inconvénient réside dans le faible rendement quantique qui nécessite l'utilisation d'un laser pour exciter les ions luminescents présents dans la matrice cristalline. D'autre part, les propriétés de luminescence sont très nettement altérées, lorsque ces particules sont utilisées directement en milieu aqueux.

Notamment, on pourra citer la demande de brevet publiée sous le numéro US 2003/0180780 qui décrit des nanoparticules d'oxyde métallique enrobé de silane pouvant servir d'agent de marquage pour des molécules d'intérêt biologique. Le seul exemple donné concerne des nanoparticules de Eu₂O₃ enrobé d'une très fine couche de silane obtenue à partir de 3-aminopropyltriméthoxysilane (APTMS), ces nanoparticules présentant un diamètre moyen compris entre 100 et 200nm.

Pour les applications biologiques, il est intéressant d'utiliser des nanoparticules de taille plus petite dont les suspensions colloïdales présentent une meilleure stabilité et dont les composants présentent une plus grande " furtivité " ou " molécularité ". L'utilisation, en tant que marqueur, de nanoparticules de taille plus petite permet un meilleur ciblage des fonctions biologiques.

Dans ce contexte, la présente invention se propose de fournir des nanoparticules sondes hybrides pour le marquage biologique, de taille suffisamment petite et présentant une stabilité améliorée, face aux agressions du milieu aqueux extérieur notamment.

L'invention a donc pour objet des nanoparticules hybrides comprenant :
- une nanosphère, de diamètre moyen compris dans la gamme allant de 2 à 9 nm, composée, à au moins 90% en poids de Ln₂O₃, avec Ln qui représente une terre rare, une terre rare dopée avec une terre rare ou un actinide, ou un mélange de terres rares, ou bien un mélange de terre rare et d'actinide dans lequel au moins 50% des ions métalliques sont des ions terre rare,

- un enrobage autour de la nanosphère, constitué majoritairement de polysiloxane fonctionnalisé, et qui présente une épaisseur moyenne comprise dans la gamme allant de 0,5 à 10 nm et, de préférence, supérieure à 2 nm et inférieure ou égale à 10 nm, et dans lequel de 5 à 75 % des atomes de silicium sont liés à quatre autres atomes de silicium par des ponts oxygène, et
- au moins un ligand biologique greffé par liaison covalente à l'enrobage de polysiloxane.

En particulier, l'utilisation de nanoparticules de sesquioxyde Ln₂O₃ dopées avec une terre rare est particulièrement avantageuse, étant donnée l'extrême finesse de leur raie d'émission et la possibilité d'obtenir des raies d'émission différentes en dopant avec des cations différents.

De plus, l'utilisation de nanoparticules rendues luminescentes par la présence d'ions terres rares (excellente photostabilité, long temps de vie de l'état excité) dans la nanosphère de coeur et de molécules luminescentes dans et/ou à la surface de l'enrobage permet des détections résolues en temps, en particulier en utilisant un outil performant pour l'imagerie optique comme la microscopie de luminescence en temps résolu.

Par ailleurs, si ces nanoparticules contiennent des lanthanides présentant des caractéristiques magnétiques intéressantes, comme le gadolinium, elles peuvent être utilisées comme agent de contraste pour l'IRM ou également dans des systèmes thérapeutiques permettant la destruction des cellules ciblées suite à l'interaction des nanoparticules avec un champs magnétique alternatif appliqué par l'extérieur, engendrant par exemple une hyperthermie.

De plus, ces nanoparticules, si elles contiennent des éléments présentant une grande capacité de capture de neutrons couplée avec une réaction fortement énergétique comme le ¹⁵⁷Gd ou ²³⁵U, peuvent être utilisées dans des thérapies (par exemple contre le cancer) basées sur la capture de neutrons.

Les suspensions colloïdales des nanoparticules ci-dessus définies font également partie de l'invention.

L'invention a également pour objet un procédé de préparation des nanoparticules hybrides telles que définies ci-dessus, éventuellement sous la forme de suspension colloïdale comprenant les étapes successives suivantes :
a) préparer une suspension colloïdale de nanosphères, de diamètre moyen compris dans la gamme allant de 2 à 9 nm, composées, à au moins 90% en poids de Ln₂O₃, avec Ln qui représente une terre rare, une terre rare dopée avec une terre rare ou un actinide, un mélange de terres rares, ou bien un mélange de terre rare et d'actinide dans lequel au moins 50% des ions métalliques sont des ions terre rare,
b) ajouter à la suspension colloïdale la quantité nécessaire d'un mélange d'organoalcoxysilane et d'agent réticulant pour former à la surface des particules un enrobage, constitué majoritairement de polysiloxane fonctionnalisé avec au moins un groupe réactif, qui présente une épaisseur moyenne comprise dans la gamme allant de 0,5 à 10 nm, et, de préférence, supérieure à 2 nm et inférieure ou égale à 10 nm, et dans lequel de 5 à 75 % des atomes de silicium sont liés à quatre autres atomes de silicium par des ponts oxygène, et
c) greffer chimiquement à l'enrobage au moins un ligand biologique par couplage avec un groupe réactif présent en surface de l'enrobage,
d) éventuellement séparation et séchage des nanoparticules hybrides obtenues.

La description ci-après, en référence aux figures annexées, permet de mieux comprendre l'objet de l'invention. Les différentes variantes décrites ci-après, lorsqu'elles ne s'excluent pas l'une l'autre, peuvent être combinées.

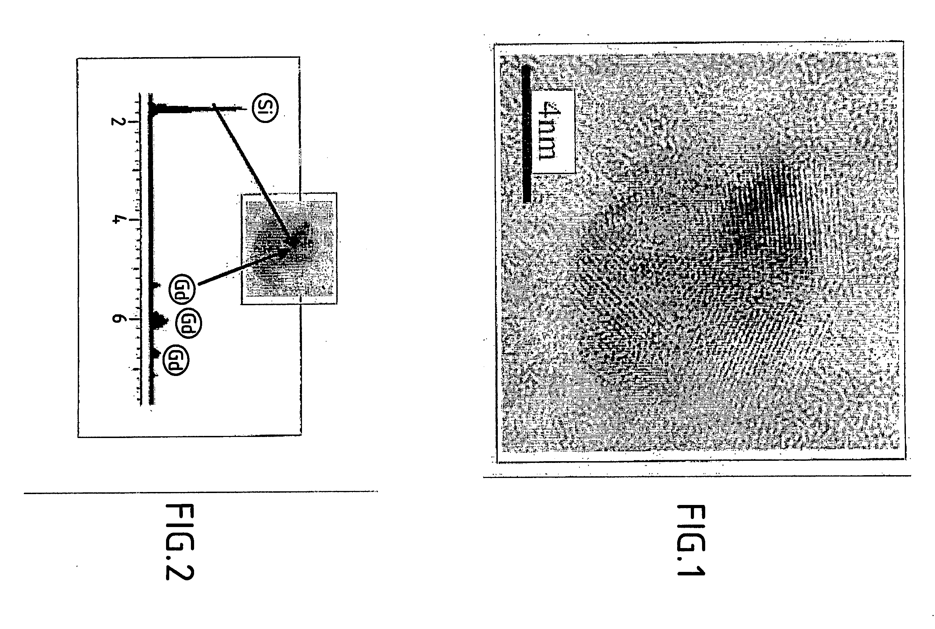

La **Fig. 1** est une image de microscopie électronique à transmission à haute résolution d'une particule de 8nm de Gd₂O₃ :Tb³⁺, enrobée d'une couche de 3,5mn d'épaisseur, obtenue selon l'exemple 4 ci-après.
La **Fig. 2** est un spectre d'analyse EDX (couplée au HRTEM), mesuré le long de la particule de 8nm de Gd₂O₃ :Tb³⁺, enrobée d'une couche de polysiloxane de 3,5nm d'épaisseur, obtenue selon l'exemple 4 ci-après.

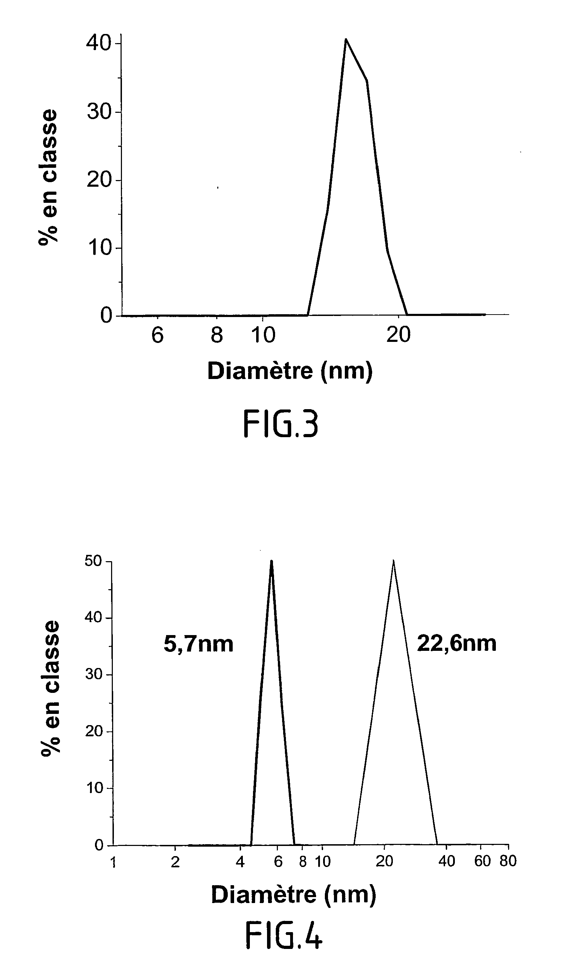

La **Fig. 3** présente les résultats de mesure de la taille des particules de 8nm après enrobage par une couche de polysiloxane de 3,5nm (exemple 4).
La **Fig. 4** présente, de façon comparée, les résultats de mesure de la taille des particules initiales (colloïde dialysé, 5,7nm) et après enrobage par une couche de polysiloxane de 8,0nm (exemple 3).

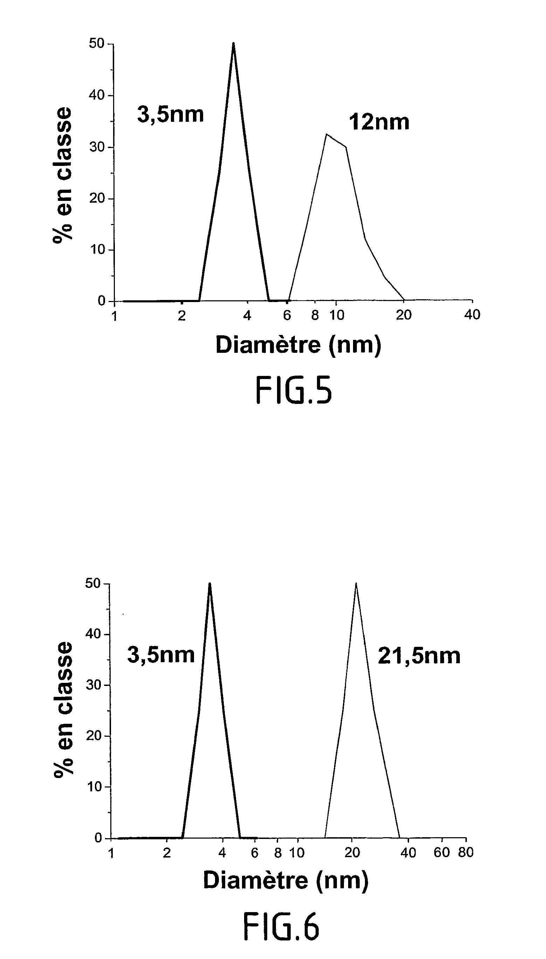

La **Fig. 5** présente, de façon comparée, les résultats de mesure de la taille des particules initiales (colloïde dialysé, 3,5nm) et après enrobage par une couche de polysiloxane de 5,0nm (exemple 1).
La **Fig. 6** présente, de façon comparée, les résultats de mesure de la taille des particules initiales (colloïde dialysé, 3,5nm) et après enrobage par une couche de polysiloxane fonctionnalisé par de la fluorescéine de 8,0nm (exemple 6).
La **Fig. 7** montre les spectres d'émission du colloïde recouvert de polysiloxane fonctionnalisé par de la fluorescéine de l'exemple 6 excité à 230nm en fonction des différentes fractions obtenues lors de la chromatographie sur colonne.
La **Fig. 8** montre la fluorescence de nanoparticules avec un coeur d'oxyde de gadolinium enrobé de polysiloxane fonctionnalisé par de la fluorescéine et par des brins d'oligonucléotide, ces nanoparticules étant immobilisées sur une biopuce par hybridation avec des brins d'oligonucléotides complémentaires. (exemple 9)
La **Fig. 9** représente le spectre d'émission du colloïde correspondant à la fraction 2 de la chromatographie sur colonne présentée **Fig. 7** et excité à 540nm après dégradation de la fluorescéine.
La **Fig. 10** présente, de façon comparée, la mesure de luminescence entre les particules du colloïde initial et dialysé de 5,7nm et celles enrobées d'une couche de polysiloxane de 8,0nm (exemple 3).
La **Fig. 11** présente, de façon comparée, la mesure de luminescence entre les particules du colloïde initial et dialysé de 3,5nm et celles enrobées d'une couche de polysiloxane de 5,0nm (exemple 1).
La **Fig. 12** présente, de façon comparée, la mesure de luminescence entre les particules du colloïde initial et dialysé de 3,5nm et celles enrobées d'une couche de polysiloxane de 8,0nm (exemple 2).
La **Fig. 13** présente les spectres d'émission du colloïde recouvert de polysiloxane de l'exemple 5 excité à 230nm en fonction de la dialyse de celui-ci dans l'éthanol et de sa dispersion dans de l'eau acidifié par HCl 0,2M.
La **Fig. 14** présente le spectre d'excitation des particules de Gd₂O₃ : Tb³⁺ (5%) (diamètre de 3,5nm) et recouvertes d'une couche de polysiloxane de 5,0nm (exemple 1).

En préambule à la description plus précise de l'invention, les définitions des termes utilisés dans la suite du mémoire descriptif sont données ci-après.

Les terres rares comprennent le scandium et l'yttrium et la série des lanthanides qui sont Ce, Pr, Nd, Pm, Sm, Eu, Gd, Tb, Dy, Ho, Er, Tm, Yb, Lu. Au sens de l'invention, par terre rare, on entend de préférence un élément choisi parmi l'yttrium et la série des lanthanides.

Les actinides sont Ac, Th, Pa, U, Np, Pu, Am, Cm, Bk, Cf, Es, Fm, Md, No et Lw.

Le diamètre moyen ou diamètre médian de nanosphères, nommé d₅₀, est défini par le diamètre en dessous duquel on trouve 50% de la masse des nanosphères et est déterminé par la technique de diffusion laser (spectroscopie de corrélation de photons).

Un sesquioxyde Ln₂O₃ est dit dopé par une terre rare ou un actinide, si celui-ci est présent à raison d'au plus 25% des cations métalliques. Au-delà, il sera question de mélange.

La présente invention concerne une sonde pour des applications biologiques comprenant essentiellement une nanosphère de coeur entourée d'un enrobage, également nommé couronne, autorisant le greffage par liaison covalente d'au moins un ligand biologique et éventuellement d'autres molécules organiques. La nanosphère est un coeur essentiellement sphérique inorganique luminescent de taille nanométrique. Plus précisément, ce coeur sphérique est constitué de Ln₂O₃ où Ln représente une terre rare pure, ou dopée avec une terre rare, un actinide ou une combinaison de ces derniers, un mélange de terres rares, ou encore un mélange de terre rare avec un actinide, dans lequel au moins 50 % des cations métalliques sont des cations terre rare. Cette nanosphère de coeur présente un diamètre moyen compris dans la gamme allant de 2 à 9 nm. Au moins 90 % du poids de la nanosphère est constitué de Ln₂O₃ ci-dessus défini, le reste pouvant notamment être constitué par l'hydroxyde correspondant.

L'enrobage est une couche intermédiaire entourant les nanosphères, à laquelle les ligands biologiques sont liés par liason covalente. L'enrobage est constitué majoritairement de polysiloxane fonctionnalisé et présente une épaisseur moyenne comprise dans la gamme allant de 0,5 à 10 nm, et de préférence supérieure à 2 nm. La fonction de cet enrobage est, sans que cette liste soit limitative, au moins triple : elle doit assurer la protection (étanchéité) du coeur vis à vis du milieu extérieur ; elle doit servir de sites de greffage pour les ligands biologiques fixés dessus ; elle doit augmenter les performances optiques du coeur minéral par transfert d'énergie de la couronne absorbant dans l'UV vers le coeur ré-émettant dans le visible.

En fonction de l'application visée, on choisira la nature de la nanosphère constituant le coeur sphérique des nanoparticules selon l'invention qui est, de façon préférée, constitué à 80 % au moins en poids, de sesquioxyde de terre rare éventuellement dopé. De préférence, cette nanosphère est dopée ou voire même codopée, c'est à dire dopée avec au moins deux éléments de nature différente.

De façon avantageuse, la nanosphère est constituée, à au moins 80 % en poids et, de préférence, à au moins 90 % en poids de Y₂O₃ ou Gd₂O₃, Gd₂O₃ étant préféré.

Lorsqu'une luminescence classique est recherchée, la nanosphère est dopée par un lanthanide de type Eu, Tb, Er, Nd, Yb, Tm représentant de 0,1 à 25% des cations métalliques de la nanosphère. Lorsqu'une luminescence dans l'infra rouge est recherchée, la nanosphère est dopée par un lanthanide de type Nd ou Yb. Lorsqu'une luminescence antistokes est recherchée, la nanosphère est dopée par un lanthanide de type Er.

Comme mentionné précédemment, pour la détection combinatoire (multiplexing), la nanosphère est dopée par au moins deux lanthanides différents représentant de 0,1 à 25 % des cations métalliques de la nanosphère, l'un au moins de ces lanthanides étant choisi parmi Eu et Tb.

Par ailleurs, pour faciliter la détection, et en particulier pour les applications dans le domaine de l'imagerie par résonance magnétique (IRM) ou les applications en thérapie, il est avantageux que la nanosphère présente, en plus de ses propriétés de luminescence, des propriétés magnétiques. Aussi, de façon avantageuse, la nanosphère contient des cations de terre rare présentant un comportement magnétique choisis parmi Gd, Nd, représentant au moins 10 % de l'ensemble des cations métalliques présents dans le coeur, si une application en imagerie médicale est envisagée, et d'au moins 50 %, si une application en hyperthermie est envisagée.

Selon une autre variante avantageuse, de 0,01 % à 50 %, et de préférence de 0,1 à 10% des cations métalliques de la nanosphère sont des cations actinide choisis parmi Ac, Th, Pa, Np, U, Pu. Un dopage de la nanosphère avec de tels radionucléides trouve notamment application en thérapie liée à des réactions nucléaires.

Une autre variante avantageuse concerne des nanoparticules hybrides dont au moins 0,1% des cations métalliques de la nanosphère sont des isotopes radioactifs de durée de demi vie inférieure à 1000 ans, ou dont au moins 1 %, de préférence au moins 5 %, des cations métalliques de la nanosphère sont des isotopes présentant une grande capacité de capture de neutrons couplée à une forte énergie de réaction (par exemple ¹⁵⁷Gd, ²³⁵U). De telles nanoparticules présentent un grand intérêt pour des systèmes de thérapie basée sur des réactions de décomposition nucléaire, par exemple pour la destruction de cellules cancéreuses.

De préférence, la nanosphère est constituée, à au moins 90 % en poids, de sesquioxyde de lanthanide éventuellement dopé. Les nanoparticules hybrides, selon l'invention dont la nanosphère est constituée, à au moins 90 % en poids, de Gd₂O₃ dopé par Tb ou Eu, sont particulièrement préférées. Gd³⁺ est en effet un cation présentant à la fois un fort paramagnétisme et pouvant servir de matrice aux cations luminescents Eu et Tb.

Les nanosphères de sesquioxyde de terre rare, et notamment de sesquioxyde de gadolinium, sont sensibles à l'hydrolyse en milieu aqueux. Pour éviter la transformation du sesquioxyde en hydroxyde, ce dernier est enrobé d'une couche protectrice constituée majoritairement de polysiloxane fonctionnalisé. Cette dernière doit être suffisamment épaisse pour assurer cette protection : son épaisseur moyenne est comprise dans la gamme allant de 0,5 à 10 nm, et de préférence est supérieure à 1 nm. La couche d'enrobage représente avantageusement au moins 20 % du volume total de la nanoparticule et de préférence de 25 à 75 % du volume total de la nanoparticule. L'épaisseur est mesurée par granulométrie laser (technique PCS). Il est également possible de retrouver, pour une nanoparticule isolée, le diamètre de la nanosphère et l'épaisseur de la couche, par microscopie électronique à transmission.

De façon avantageuse, cet enrobage protège les propriétés de luminescence du coeur d'oxyde et/ou autorise un transfert d'énergie par rapport au coeur sphérique, c'est à dire que l'excitation UV est absorbée par la couronne de polysiloxane et transférée au coeur sphérique ce qui augmente sa luminescence. Cette fonction est particulièrement assurée, si 5 à 75 %, de préférence 30 à 50 % des atomes de silicium de la couche de polysiloxane sont liés chacun à quatre autres atomes de silicium par des ponts oxygène, ce qui constitue donc une variante préférée de l'invention. Par conséquent, selon une variante avantageuse de l'invention, 25 à 95 % et, de préférence, de 50 à 70% des atomes de silicium de l'enrobage de polysiloxane fonctionnalisé sont liés de façon covalente à un atome de carbone et sont donc fonctionnalisés.

Par ailleurs, la densité de cette couche de polysiloxane fonctionnalisé peut être choisie en fonction de l'effet privilégié que l'on souhaite lui attribuer. Notamment, une couche présentant une densité comprise dans la gamme allant de 1,6 à 2,4 et, de préférence, comprise dans la gamme allant de 1,8 à 2,1 est préférée pour favoriser encore la protection du coeur sphérique contre l'hydroxylation et conserver sa luminescence. Un enrobage présentant une densité inférieure à 2 est préférée pour les applications en IRM ou en hyperthermie.

Cette couche de polysiloxane fonctionnalisé permet également le greffage par liaison covalente d'au moins un ligand biologique et éventuellement d'autres molécules organiques ou polymères. En particulier, entre 10 et 100 000 molécules organiques luminescentes, notamment choisies parmi les dérivés de la rhodamine ou de la fluorescéine, sont greffées à la surface et/ou au sein de l'enrobage, en plus du ou des ligands biologiques, ce qui permet une détection résolue en temps. En effet, le temps de vie de l'état excité est de quelques nanosecondes pour les molécules organiques luminescentes et de l'ordre de la microseconde pour les nanoparticules de sesquioxyde de lanthanide.

Selon une variante de l'invention, 1 à 1000 et, de préférence, 1 à 100 molécules de ligand biologique sont greffées à la couche de polysiloxane fonctionnalisé par liaison covalente. Selon une autre variante de l'invention, moins de 10% en poids de ces nanoparticules comprennent plus de deux molécules de ligand biologique greffées sur la couche intermédiaire.

Par ligand biologique, on entend un composé qui possède au moins un site de reconnaissance lui permettant de réagir avec une molécule cible d'intérêt biologique. Les ligands biologiques greffés sont, par exemple, dérivés de nucléotides, sucres, vitamines, hormones, biotine, streptavidine, ou toute autre molécule organique présentant un intérêt pour la vectorisation biologique.

Il est également possible que des molécules luminescentes ou molécules complexantes, autres que le(s) ligands biologiques, soient greffées sur la couche de polysiloxane fonctionnalisé. Des molécules polaires ou chargées du type organophosphonates, amines quaternaires, peuvent également être greffées sur la couche intermédiaire. Une autre possibilité est de greffer, sur la couche de polysiloxane, des molécules de polymère solubles dans l'eau de masse moléculaire inférieure à 5000 g/mol et de préférence inférieure à 1000, par exemple, tel que le polyéthylène glycol ou le dextran.

La présente invention vise également un procédé de préparation de nanoparticules hybrides telles que définies ci-dessus, éventuellement sous la forme de suspension colloïdale. Ce procédé comprend différentes étapes successives détaillées ci-après.

La première étape du procédé réside dans l'obtention en grande quantité de nanosphères cristallines de diamètre moyen compris entre 2 et 9 nm, obtenues avec une faible polydispersité et facilement redispersables, c'est à dire sans la formation d'agglomérat. Le procédé selon l'invention passe par la préparation directe d'une dispersion colloïdale isodisperse. Par dispersion isodisperse, on entend une dispersion dont la distribution en taille mesurée par spectroscopie de corrélation de photons est très resserrée, c'est à dire, par exemple, que plus de 90% des particules ont un diamètre compris entre le diamètre moyen de la dispersion plus ou moins 20% du d₅₀. Plus la distribution en diamètre est resserrée, plus le système est appelé isodisperse ou monodisperse.

Ces nanoparticules sont préparées selon la méthode polyol. Cette méthode « polyol » consiste en une précipitation directe d'un solide au sein d'un polyalcool porté à une température comprise généralement entre 130°C et 250°C. Un précurseur métallique est préalablement dissous dans le polyol (par exemple du diéthylène glycol), puis après ajout éventuel d'un réactif, la solution est portée à une température supérieure à 150°C. Le polyol agit comme un stabilisant, limite la croissance des particules et minimise l'agglomération des poudres.

Avec cette méthode, différentes poudres submicroniques d'oxydes ont déjà été synthétisées, on peut ainsi citer CeO₂, LaPO₄ dopé Ce³⁺ (C. Feldmann, Advanced Materials, 13 (2003) 101), Y₂O₃ dopé Eu³⁺ (C. Feldmann, J. Merikhi, Journal of Materials Science, 38 (2003) 1731-1735) et Eu₂O₃, Gd₂O₃ dopé Eu³⁺ et Y₂O₃ dopé Eu³⁺ (R. Bazzi et al J. of Colloid and Interface Science, 273 (2004), 191-197). On peut également citer Bazzi R et al. J. of Luminescence, Amsterdam, vol 102-103, mai 2003, 445-450. Les nanosphères utilisées dans la présente invention sont, par exemple, obtenues selon la méthode « polyol » décrite dans Journal of Luminescence, 102-103 (2003) 445-450, et Journal of Colloid and Interface Science*,* doi :10.1016/j.jcis.2003.10.031. Sans que cette liste soit limitative, les précurseurs utilisés peuvent être des chlorures, des nitrates ou des acétates. Des concentrations supérieures au g/l peuvent ainsi être obtenues. De façon avantageuse, on obtient une suspension colloïdale contenant entre 100 mg et 100 g de nanosphères par litre de solvant.

La synthèse des nanosphères de sesquioxyde est, par exemple, réalisée à une température inférieure à 200°C dans un solvant polaire tel qu'un polyol, du type diéthylène glycol (DEG) dont le fort pouvoir solvatant permet un contrôle de la taille des particules. Des précurseurs des différents sesquioxydes métalliques qui vont constituer les nanosphères, par exemple, des chlorures de terre rare TRCl₃ (TR : Gd, Y) et des chlorures de lanthanide Ln'Cl₃ (Ln' : Eu, Tb, Er, Nd, Yb, Tm) ou des nitrates d'actinides (U) sont dispersés dans le diéthylène glycol. La concentration globale en ions métalliques est, avantageusement, comprise entre 0,1 et 0,5 M et la quantité relative de chlorures de terre rare TRCl₃ (TR : Gd, Y) et de chlorures de lanthanide Ln'Cl₃ (Ln' : Eu, Tb, Er, Nd, Yb, Tm) ou de nitrates d'actinides (U) dépend du taux de dopage ou de la nature du mélange désiré. Après agitation, une solution aqueuse d'hydroxyde de sodium, avantageusement 1ml, est ajouté et le mélange est chauffé dans un bain d'huile silicone à 140°C pendant une heure. Après dissolution complète des composés présents, la solution est portée à 180°C pendant 4 heures sous agitation vigoureuse. Il en résulte une solution colloïdale, stable pendant plusieurs mois, de nanosphères dispersées dans le DEG. Quelle que soit la quantité de dopant (terre rare ou actinide), les particules de taille nanométrique se présentent sous forme de solution solide constituée d'une seule phase. La concentration des ions et le taux d'hydrolyse (rapport de la quantité d'eau ajoutée et de la quantité d'ions métalliques) peut être ajustée, afin de contrôler la taille. Il s'avère que la concentration en OH⁻ doit être la plus faible possible pour éviter la précipitation d'hydroxydes. L'addition d'un équivalent de NaOH par rapport au nombre de moles de cations terre rare semble un bon compromis : une plus faible quantité est caractérisée par un faible rendement (moins de 30 % d'oxyde de lanthanide) et une plus importante aboutit à une précipitation irréversible d'hydroxydes.

Par conséquent, de façon particulièrement préférée, à l'étape a) du procédé, une suspension colloïdale contenant entre 100 mg et 100 g de nanosphères par litre de solvant est préparée par dissolution de précurseurs de terre rare et/ou d'actinides, dans un solvant polaire, en particulier un polyol du type éthylèneglycol et chauffage à une température comprise entre 130 et 250°C, en présence de la quantité d'eau au moins nécessaire pour la formation du sesquioxyde désiré et éventuellement d'une base telle que NaOH à une concentration comprise entre 0,01 et 1 mol/l de solvant.

La suspension colloïdale de nanosphères obtenue peut, éventuellement, être purifiée et/ou concentrée, en éliminant les sels solubles résiduels, une partie du polyol, par exemple par évaporation sous pression réduite, ou en changeant de solvant, par exemple par chromatographie ou dialyse, et contrôler la quantité d'eau présente dans la solution.

Au sens de l'invention, les nanosphères de Ln₂O₃ sont ensuite enrobées par une couche de molécules capables d'établir des interactions fortes et durables à la fois avec la surface de sesquioxydes et avec des molécules organiques (fluorophores, dispersants...) et/ou des ligands biologiques qui vont y être fixés. D'autre part, cette couche assure une protection des nanosphères lorsqu'elles sont dispersées en milieu aqueux. En effet, une dissolution des nanosphères et/ou une chute de l'intensité de luminescence est généralement observée lorsque ces nanosphères sont dispersées dans l'eau. Pour cela, des formateurs de réseaux, et non des modificateurs (qui ne permettent pas de former une couche réticulée autour de la nanosphère) sont utilisés. Les formateurs de réseaux sont des composés qui, outre le ou les liens qu'ils établissent avec la surface de sesquioxyde de terre rare, sont capables de réagir entre eux (par réaction de condensation entre les plus proches voisins) pour constituer un véritable réseau emprisonnant la nanosphère. Ces composés sont en général des organoalcoxysilanes de formule RₓSi(OR')_{y} avec y=2, 3 ; x = 4-y et R choisi de façon à pouvoir immobiliser une molécule organique active. Afin d'obtenir un réseau dense et robuste, RₓSi(OR')_{y} est mélangé avec un agent réticulant tel que le tétraéthylorthosilicate (TEOS). La couche de polysiloxane, également nommée enrobage ou couronne, peut être, en particulier, obtenue par hydrolyse - condensation d'un mélange d'organotrialcoxysilane choisi parmi l'aminopropyltriéthoxysilane (APTES), glycidyloxypropyltriéthoxysilane (GPTES), mercaptopropyltriéthoxysilane (MPTES) et isocyanatopropyltriéthoxysilane (IPTES)) avec un agent réticulant, tel que le TEOS, en présence des nanosphères de sesquioxyde Ln₂O₃ telles que précédemment obtenues. Le rapport Si/C est contrôlé par la quantité d'agent réticulant, le plus souvent de TEOS, introduite. La quantité d'organoalcoxysilane/agent réticulant ajoutée dépend du nombre de nanosphères dispersées en solution, de leur taille (et donc de leur surface) et de l'aire qu'occupe une molécule adsorbée. La présence d'une base de type triméthylamine est préférable, de préférence entre 0,01 et 0,1 mol/l. L'utilisation d'organotrialcoxysilane permet d'introduire dans la couronne des groupes réactifs qui permettront l'accrochage de molécules actives (ligand biologique, fluorophores). Aussi, pour permettre ce couplage, certains des précurseurs de type organoalcoxysilane de la couche de polysiloxane portent des groupements réactifs tels que des groupements carboxylique, amine, aldéhyde, ou époxyde. L'hydrolyse-condensation de ces précurseurs fournit une couche de polysiloxane fonctionalisé capable de réagir avec un ligand biologique ou toute autre molécule organique ou polymère à greffer. Le TEOS assure la réticulation du polysiloxane formé, dans lequel la quantité d'atomes de silicium liés uniquement à des atomes d'oxygène (noeuds de réticulation), c'est-à-dire qui ne proviennent que de TEOS, est compris entre 5 et 75 %, de préférence entre 30 et 50 %.

Les molécules non adsorbées et les produits secondaires sont, généralement, extraits de la solution colloïdale par dialyse contre l'éthanol, de l'eau ou un mélange équivolumique éthanol/eau.

On peut détailler, sans caractère limitatif, un procédé de formation de l'enrobage de polysiloxane se déroulant, avantageusement, en quatre phases. La première consiste à additionner à 5 ml de nanosphères de Ln₂O₃ dispersées dans une solution de DEG contenant n_{APTES(1)} mol d'APTES tel que 4 < 100 x n_{APTES(1)}/(n_{APTES(1)}+ n_{APTES(2)}+n_{TEOS}) < 16. Après au moins 20 minutes d'agitation, 3n_{APTES(1)} mol d'eau diluée dans le DEG contenant 0,1 M de triéthylamine (Et₃N) sont ajoutés (2^{ème} phase). La solution est agitée pendant une heure avant d'ajouter au mélange précédent (3^{éme} phase) n_{APTES(2)}=5n_{APTES(1)} mol d'APTES et n_{TEOS} mol de TEOS tel que 5 < 100 x n_{TEOS}/(n_{APTES(1)}+ n_{APTES(2)}+n_{TEOS}) < 75. Enfin, après 20 minutes, (15n_{APTES(1)} + 4n_{TEOS}) mol d'eau diluée dans le DEG contenant 0,1 M de triéthylamine (Et₃N) sont ajoutés au mélange. Le mélange est alors agité pendant 48 heures à 20°C < T < 40°C.

Selon un mode de réalisation particulier, l'étape c) peut être précédée d'une étape de greffage de molécules luminescentes et/ou de molécules complexantes et/ou de molécules polaires ou chargées et/ou de molécules de polymères solubles dans l'eau, par couplage avec un groupe réactif présent en surface de l'enrobage.

En plus des ligands biologiques, l'enrobage de polysiloxane fonctionnalisé peut permettre le greffage covalent de molécules organiques luminescentes, dérivés de la fluorescéine et de la rhodamine notamment. La fonctionnalisation par ces fluorophores peut être réalisée selon au moins deux manières différentes. La première consiste à modifier des molécules d'organoalcoxysilane par ces fluorophores avant la réaction d'hydrolyse. Par exemple, 5 à 30 % des molécules d'organoalcoxysilane, notamment d'APTES introduites lors de la première ou de la deuxième phase ci-dessus mentionnées, sont couplées, au préalable, à la fluorescéine isothiocyanate (FTIC) par réaction entre la fonction amine d'APTES et la fonction isothiocyanate de FTIC. Les précurseurs de polysiloxane ainsi modifiés sont alors ajoutés à un mélange d'organoalcoxysilane et de TEOS pour former la couronne. La seconde méthode consiste à greffer les fluorophores après formation de la couche de polysiloxane par condensation entre les groupes réactifs portés par les fluorophores et ceux présents dans la couche de polysiloxane.
Les ligands biologiques doivent être accessibles pour des réactions ultérieures de couplage sonde/cible et sont, donc, quant à eux, greffés chimiquement sur la couche de polysiloxane, par couplage classique avec des groupes réactifs présents, éventuellement précédé d'une étape d'activation. Les réactions assurant le couplage sont connues et décrites, par exemple, dans « Bioconjugatre Techniques », G. T. Hermanson, Academic Press, 1996 ou dans « Fluorescent and Luminescent Probes for Biological Activity. A Practical Guide to Technology for Quantitative Real-Time Analysis », Second Edition, W. T. Mason, ed., Academic Press, 1999. Par exemple, le couplage est effectué par addition d'une solution aqueuse de ligands biologiques dont la quantité est au moins supérieure au nombre de sites de greffage (correspondant aux groupes réactifs) sur l'enrobage. Dans le cas où la couche de polysiloxane est fonctionnalisée avec des groupes amine, des biomolécules portant au moins une fonction -COOH peuvent être greffées. Cependant il est nécessaire d'activer au préalable la fonction -COOH de la biomolécule par un mélange d'EDC et de NHS .

Après greffage des ligands biologiques, les produits secondaires en excès sont éliminés par dialyse ou par chromatographie sur colonne. Cette dernière technique est utilisée seulement lorsque les nanoparticules sont fonctionnalisées par des dérivés de la fluorescéine. Afin d'éviter la dénaturation par la répétition des étapes de greffage et de purification, le greffage des ligands biologiques est, de préférence, réalisé en dernier.

Les nanoparticules selon l'invention trouvent différentes applications. On peut notamment citer leur utilisation pour la détection de biomolécules (biopuces), le ciblage de substrats biologiques (imagerie IRM), pour la destruction de cellules ou de tumeurs solides en thérapie (hypertthermie, capture de neutrons).

Les exemples ci-après sont donnés à titre purement illustratif et n'ont pas de caractère limitatif.

### Exemple 1 :

Un colloïde de Gd₂O₃ dopé à 5% en Tb³⁺ est préparé par dissolution d'une quantité de 50g.l⁻¹ de sels de chlorures de gadolinium et de terbium dans un volume de 20ml de diéthylène glycol. La taille finale des particules, confirmée par HRTEM et mesurée par granulométrie laser, est de 3,5nm. Le colloïde est dialysé pendant 24h, à chaud (40°C), sous agitation dans du diéthylène glycol. Le rapport volume de diéthylène glycol propre / volume de colloïde à dialyser est de 20.

Autour de ces particules, une couche de polysiloxane fonctionnalisé d'une épaisseur de 5,0nm est synthétisée par voie sol-gel. Dans une solution contenant 5,0ml de colloïde dialysé (4,53.10¹⁹particules.l⁻¹), dissolution de 28,4g.l⁻¹ d'aminopropyltriethoxysilane (APTS), 17,6g.l⁻¹ de tétraéthyl orthosilicate (TEOS) et 13,2ml par litre d'une solution aqueuse de 0,1M de triéthylamine.

La réaction s'effectue à 40°C dans un bain d'huile et sous agitation. Elle comporte plusieurs étapes :
à t = 0 h ajout de m₁ correspondant à 16% de la masse totale d'APTS
à t = 0,33 h hydrolyse de l'APTS par ajout de 10% du volume d'eau total
à t = 1,33 h ajout de m₂ correspondant à la masse d'APTS restant et de la totalité du TEOS
à t = 2,33 h ajout des 90% du volume d'eau restante
à t = 50,33 h, fin de la synthèse.

### Exemple 2 :

Un colloïde de Gd₂O₃ dopé à 5% en Tb³⁺ est préparé dans les mêmes conditions que celles de l'exemple 1.

Autour de ces particules, une couche de polysiloxane fonctionnalisé d'une épaisseur de 8,0nm est synthétisée par voie sol-gel. Dans une solution contenant 5,0ml de colloïde dialysé (4,53.10¹⁹particules.l⁻¹), on dissout 86,4g.l⁻¹ d'APTS, 54,2g.l⁻¹ de TEOS et 40ml par litre d'une solution aqueuse de 0,1M de triéthylamine.

La réaction de recouvrement par voie sol-gel a été réalisée dans les mêmes conditions que celles de l'exemple 1.

### Exemple 3 :

Un colloïde de Gd₂O₃ dopé à 5% en Tb³⁺ est préparé dans les mêmes conditions que celles de l'exemple 1, correspondant à une taille de 3,5nm. Des particules d'une taille de 5,7nm et de même composition ont été obtenues par ajout, dans le colloïde obtenu précédemment, d'une quantité adéquate de sels de chlorures de gadolinium et de terbium.

Autour de ces particules, une couche de polysiloxane fonctionnalisé d'une épaisseur de 8nm est synthétisée par voie sol-gel. Dans une solution contenant 5,0ml de colloïde dialysé (5,66.10¹⁸particules.l⁻¹), on dissout 58,1g.l⁻¹ d'APTS, 36,4g.l⁻¹ de TEOS et 28,4ml par litre d'une solution aqueuse de 0,1M de triéthylamine.

La réaction de recouvrement par voie sol-gel a été réalisée dans les mêmes conditions que celles de l'exemple 1.

### Exemple 4 :

Un colloïde de Gd₂O₃ dopé à 5% en Tb³⁺ est préparé dans les mêmes conditions que celles de l'exemple 3, correspondant à une taille de 5,7nm. Des particules d'une taille de 8nm et de même composition ont été obtenues par ajout, dans le colloïde obtenu précédemment, d'une quantité adéquate de sels de chlorures de gadolinium et de terbium.

Autour de ces particules, une couche de polysiloxane fonctionnalisé d'une épaisseur de 3,5nm est synthétisée par voie sol-gel. Dans une solution contenant 5,0ml de colloïde dialysé (5,65.10¹⁸particules.l⁻¹), on dissout 20,6g.l⁻¹ d'APTS, 13,0g.l⁻¹ de TEOS et 10ml par litre d'une solution aqueuse de 0,1M de triéthylamine.

La réaction de recouvrement par voie sol-gel a été réalisée dans les mêmes conditions que celles de l'exemple 1.

### Exemple 5 :

Un colloïde de Gd₂O₃ dopé à 5% en Tb³⁺ est préparé dans les mêmes conditions que celles de l'exemple 1.

Autour de ces particules, une couche de polysiloxane fonctionnalisé d'une épaisseur de 5,0nm est élaborée par voie sol-gel. Dans une solution contenant 2,5ml de colloïde dialysé (4,53.10¹⁹particules.l⁻¹) et 2,5ml de diéthylène glycol propre, on dissout 28,4g.l⁻¹ d'APTS, 17,6g.l⁻¹ de TEOS et 13,2ml par litre d'une solution aqueuse de 0,1M de triéthylamine.

La réaction de recouvrement par voie sol-gel a été réalisée dans les mêmes conditions que celles de l'exemple 1.

### Exemple 6 :

Un colloïde de Gd₂O₃ dopé à 5% en Tb³⁺ est préparé dans les mêmes conditions que celles de l'exemple 1.

Autour de ces particules, une couche de polysiloxane fonctionnalisé d'une épaisseur de 8 nm est élaborée par voie sol-gel. Dans une solution contenant 2,5ml de colloïde dialysé (2,26.10¹⁹particules.l⁻¹) et 2,5ml de diéthylène glycol propre, on dissout 9,4g.l⁻¹ d'APTS, 6,4g.l⁻¹ de TEOS et 4,4ml par litre d'une solution aqueuse de 0,1M de triéthylamine.

Six heures avant l'ajout de la masse m₂ d'APTS, celle-ci est mélangée sous agitation magnétique, à 1,4g.l⁻¹ de fluorescéine isothiocyanate (FTIC), molécule fluoresecente. Par la suite, la réaction de recouvrement a été réalisée dans les même conditions que celles de l'exemple 1, mis à part que la masse m₂ d'APTS est combiné avec la FTIC.

### Exemple 7 : greffage de l'oligonucléotide

A une solution de 100 µM d'oligonucléotides d(T)22 terminée par une fonction -COOH, est ajoutée une solution aqueuse d'EDC 0,2 M et de NHS 0,2 M. Après une heure d'agitation, 500 µl de solution aqueuse de nanoparticules d'oxyde de gadolinium enrobées d'une couche de polysiloxane fonctionnalisé (préparées selon l'exemple 1 et purifiées par dialyse) et 100 µl d'une solution tampon carbonate (0,1M ; pH 11) sont ajoutés à la solution renfermant les brins d'oligonucléotide. Au bout de deux heures d'agitation, la solution gélifie. Après filtration sur membrane, le gel est redispersé dans 200 µl d'eau milli-Q.

### Exemple 8 :

Un colloïde de Gd₂O₃ dopé à 1% en uranium est préparé par dissolution d'une quantité de 50g.l⁻¹ de sels de chlorure de gadolinium et de nitrate d'uranium dans un volume de 20ml de diéthylène glycol. La taille finale des nanosphères, confirmée par HRTEM et mesurée par granulométrie laser, est de 3,5nm. Le colloïde est dialysé pendant 24h, à chaud (40°C), sous agitation dans du diéthylène glycol. Le rapport volume de diéthylène glycol propre / volume de colloïde à dialyser est de 20.

Autour de ces nanosphères, une couche de polysiloxane fonctionnalisé d'une épaisseur de 5,0nm est synthétisée par voie sol-gel. Dans une solution contenant 5,0ml de colloïde dialysé (4,53.10¹⁹particules.l⁻¹), dissolution de 28,4g.l⁻¹ d'aminopropyltriéthoxysilane (APTS), 17,6g.l⁻¹ de tétraéthyl orthosilicate (TEOS) et 13,2ml par litre d'une solution aqueuse de 0,1M de triéthylamine.

La réaction s'effectue à 40°C dans un bain d'huile et sous agitation. Elle comporte plusieurs étapes :
à t = 0 h ajout de m₁ correspondant à 16% de la masse totale d'APTS
à t = 0,33 h hydrolyse de l'APTS par ajout de 10% du volume d'eau total
à t = 1,33 h ajout de m₂ correspondant à la masse d'APTS restant et de la totalité du TEOS
à t = 2,33 h ajout des 90% du volume d'eau restante
à t = 50,33 h, fin de la synthèse.

### Exemple 9 :

Un colloïde de Gd₂O₃ dopé à 5% en Tb³⁺ et 5% en Eu³⁺ en moles est préparé par dissolution d'une quantité de 50g.l⁻¹ de sels de chlorure de gadolinium, de terbium et d'europium dans un volume de 50ml de diéthylène glycol (2,260g de GdCl₃,6H₂O, 0,1273g de EuCl₃,6H₂O et 0,1250g de TbCl₃,6H₂O). Après ajout de 3ml d'une solution aqueuse de NaOH 2N à 70°C, chauffage à 140°C pendant 1h, puis pendant 4h à 180°C, la taille finale des particules, confirmée par HRTEM et mesurée par granulométrie laser, est de 3,5nm. Le colloïde est dialysé pendant 24h, à chaud (40°C), sous agitation dans du diéthylène glycol. Le rapport volume de diéthylène glycol propre / volume de colloïde à dialyser est de 20.

La réaction d'enrobage par voie sol-gel est réalisée dans les mêmes conditions que celles de l'exemple 1.

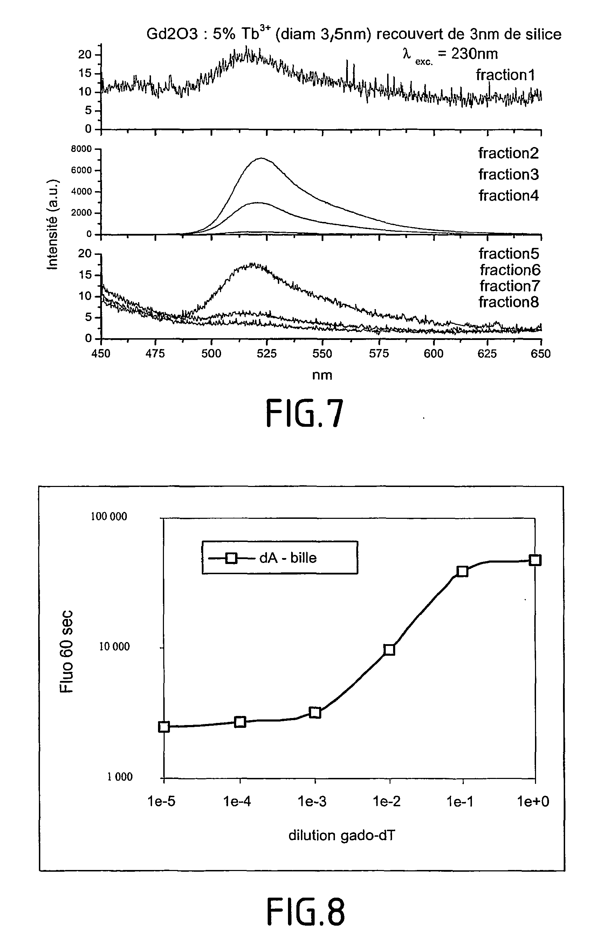

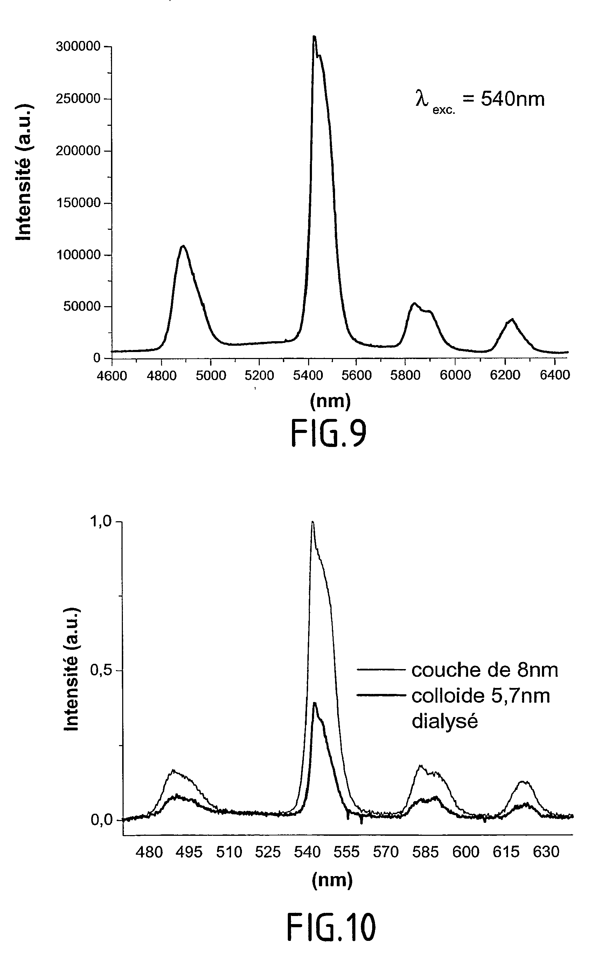

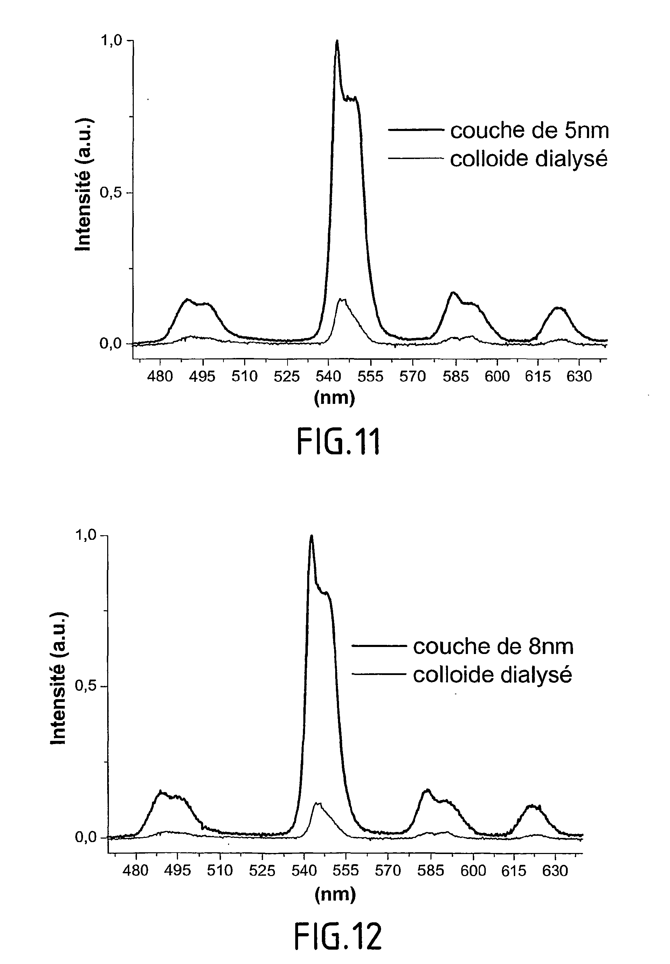

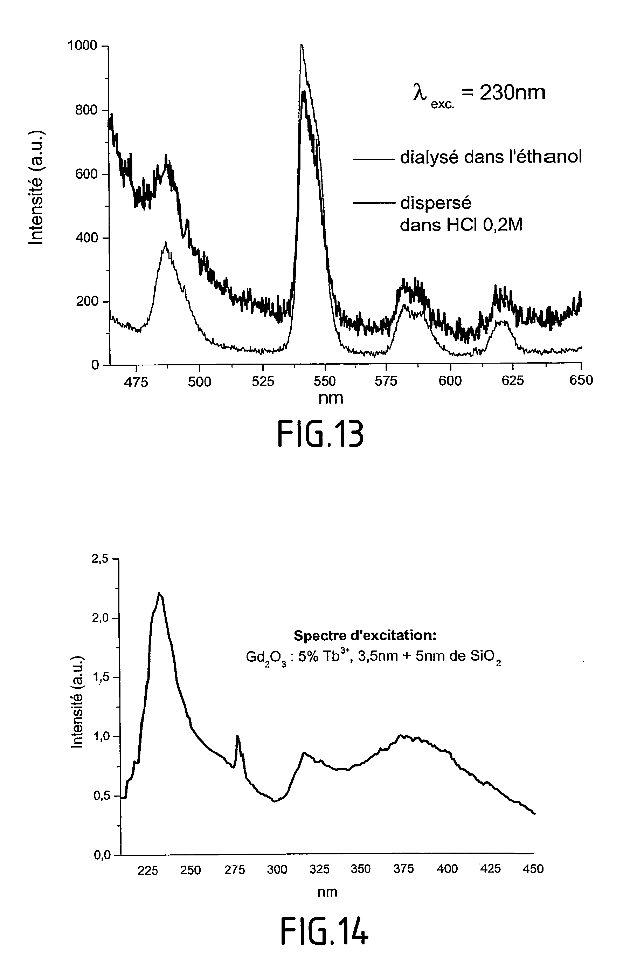

Afin de tester la possibilité de détection résolue en temps (et accessoirement faciliter la manipulation de ces nanoparticules), 10 % de l'aminopropyltrialcoxysilane sont fonctionnalisés par un colorant organique (fluorescéine) (**Fig. 7**). En outre, sa présence facilite la purification des nanoparticules hybrides par chromatographie car son avancement peut aisément être suivi à l'oeil nu. L'analyse des fractions recueillies par spectrophotométrie UV-visible et par spectroscopie de corrélation de photons confirme l'impression visuelle et permet d'identifier les fractions (2 et 3 de la **Fig. 7**) contenant les nanoparticules hybrides. La chromatographie est réalisée à l'aide d'une colonne remplie d'un gel de silice. Le volume de l'échantillon à purifier est fixé à 2,5 ml et il est élué par une solution aqueuse tamponnée, par de l'éthanol ou un mélange équivolumique eau - éthanol. Les nanosphères de Gd₂O₃ dopé Tb enrobées d'une couche de polysiloxane dont 10% des fonctions amines sont liées à un dérivé de la fluorescéine peuvent être utilisées comme marqueur pour la détection de ligands biologiques (et en particulier d'oligonucléotides). En effet, en raison des propriétés de luminescence du terbium et de la fluorescéine, ces nanoparticules, lorsqu'elles sont fonctionnalisées par une molécule sonde, permettent de marquer sélectivement des molécules biologiques en raison d'interaction spécifiques avec les sondes greffées sur les nanoparticules et donc de les détecter. En l'occurrence, le greffage d'un oligonucléotide modifié par une fonction acide carboxylique sur la nanoparticule grâce à la condensation du - COOH activé avec une des fonctions amines présentes dans le réseau de polysiloxane permet l'immobilisation de la nanoparticule sur un support solide s'il est recouvert en certains points par l'oligonucléotide complémentaire (hybridation). A la suite d'une excitation électromagnétique adaptée, la fluorescéine émet un signal lumineux permettant de repérer les endroits du support où la nanoparticule est immobilisée (**Fig. 8**). Connaissant la séquence de l'oligonucléotide sur le support et en vertu du principe de la complémentarité des bases qui préside à l'hybridation des brins d'oligonucléotides, la séquence de l'oligonucléotide greffé sur la nanoparticule peut alors être déduite. L'émission des ions Tb³⁺ est observée après destruction par irradiation laser de la fluorescéine (**Fig. 9**). Le réseau de polysiloxane fonctionnalisé qui enrobe la nanosphère de sesquioxyde permet, parallèlement au greffage de molécules actives, d'amplifier le signal de luminescence du dopant présent dans le coeur de sesquioxyde. En effet, pour un même nombre de nanoparticules dont le coeur de sesquioxyde est identique, l'intensité de la luminescence des ions Tb³⁺ est beaucoup plus importante lorsque la nanosphère de Ln₂O₃ est enrobée par une couche de polysiloxane comparée à des nanosphères non enrobées (**Fig. 10-12**). De plus, la couche de polysiloxane assure une protection efficace vis-à-vis d'une solution aqueuse d'acide chlorhydrique diluée. En effet, les spectres enregistrés à partir de deux échantillons contenant la même quantité de nanoparticules identiques, mais dispersées pour les uns dans de l'éthanol et pour les autres dans de l'HCl 0,2 M, montrent que les nanosphères enrobées par le polysiloxane fonctionnalisé sont insensibles à la présence d'eau (contrairement aux nanosphères nues) (Fig. 13). La stabilité en milieu aqueux de la nanopshère et de ses propriétés de luminescence est indispensable dans l'optique du marquage biologique. Ces résultats confirment le potentiel des nanoparticules selon l'invention et en particulier de celles de sesquioxyde de gadolinium pour le marquage et la détection de ligands biologiques dont la présence peut être révélée par :
- la fluorescence de molécules de colorant organique greffées sur la couche de polysiloxane qui enrobe la nanosphère de Ln₂O₃
- la radiation électromagnétique émise par les dopants incorporés dans la nanosphère et dont la nature peut varier au sein d'une même nanosphère (multidopage).
- des mesures magnétiques, dans la cas de nanosphère à base de sesquioxyde de gadolinium, notamment, qui est paramagnétique.

## Revendications

1. Nanoparticules hybrides comprenant :
- une nanosphère, de diamètre moyen compris dans la gamme allant de 2 à 9 nm, composée, à au moins 90% en poids de Ln₂O₃, avec Ln qui représente une terre rare, une terre rare dopée avec une terre rare ou un actinide, un mélange de terres rares, ou bien un mélange de terre rare et d'actinide dans lequel au moins 50% des ions métalliques sont des ions terre rare,
- un enrobage autour de la nanosphère, constitué majoritairement de polysiloxane fonctionnalisé, et qui présente une épaisseur moyenne comprise dans la gamme allant de 0,5 à 10 nm et dans lequel de 5 à 75 % des atomes de silicium sont liés à quatre autres atomes de silicium par des ponts oxygène, et,
- au moins un ligand biologique greffé par liaison covalente à l'enrobage de polysiloxane.

2. Nanoparticules selon la revendication 1 **caractérisées en ce que** l'enrobage présente une densité comprise dans la gamme allant de 1,6 à 2,4.

3. Nanoparticules selon la revendication 1 **caractérisées en ce que** l'enrobage présente une densité inférieure à 2.

4. Nanoparticules selon l'une des revendications 1 à 3 **caractérisées en ce que** entre 10 et 100 000 molécules organiques luminescentes sont greffées, par liaison covalente, à l'enrobage.

5. Nanoparticules selon la revendication 4 **caractérisées en ce que** les molécules organiques fluorescentes sont choisies parmi les dérivés de la rhodamine, ou de la fluorescéine.

6. Nanoparticules selon l'une des revendications 1 à 5 **caractérisées en ce que** la nanosphère est constituée, à au moins 80 % en poids, de sesquioxyde de terre rare ou de sesquioxyde de terre rare dopée.

7. Nanoparticules selon la revendication 6 **caractérisées en ce que** la nanosphère est constituée, à au moins 80 % en poids, de Gd₂O₃.

8. Nanoparticules selon la revendication 6 **caractérisées en ce que** la nanosphère est constituée, à au moins 80 % en poids, de Y₂O₃.

9. Nanoparticules selon l'une des revendications 1 à 8 **caractérisées en ce que** la nanosphère est dopée par un lanthanide de type Eu, Tb, Er, Nd, Yb, Tm représentant de 0,1 à 25% des cations métalliques.

10. Nanoparticules selon la revendication 9 **caractérisées en ce que** la nanosphère est dopée par un lanthanide de type Nd ou Yb.

11. Nanoparticules selon la revendication 9 **caractérisées en ce que** la nanosphère est dopée par un lanthanide de type Er.

12. Nanoparticules selon l'une des revendications 1 à 8 **caractérisées en ce que** la nanosphère est dopée par au moins deux lanthanides différents représentant de 0,1 à 25% des cations métalliques, l'un au moins de ces lanthanides étant choisi parmi Eu et Tb.

13. Nanoparticules selon l'une des revendications 1 à 6 **caractérisées en ce que** plus de 10% des cations métalliques de la nanosphère sont des cations de lanthanide présentant un comportement magnétique, choisie parmi Gd, Nd.

14. Nanoparticules selon l'une des revendications 1 à 6 **caractérisées en ce que** plus de 50% des cations métalliques de la nanosphère sont des cations de lanthanide présentant un comportement magnétique, choisie parmi Gd, Nd.

15. Nanoparticules selon l'une des revendications 1 à 6 **caractérisées en ce que** de 0,01% à 50% des cations métalliques de la nanosphère sont des cations d'actimide choisis parmi Ac, Th, Pa, U, Np, Pu.

16. Nanoparticules selon l'une des revendications 1 à 15 **caractérisées en ce que** au moins 1 % des cations métalliques de la nanosphère présentent une grande capacité de capture de neutron, choisis parmi les isotopes ¹⁵⁷ Gd et ²³⁵U.

17. Nanoparticules selon l'une des revendications 1 à 16 **caractérisées en ce que** de 1 à 1000 molécules de ligand biologique sont greffées à l'enrobage par liaison covalente.

18. Nanoparticules selon l'une des revendications 1 à 16 **caractérisées en ce que** moins de 10% en poids de ces nanoparticules comprennent plus de deux molécules de ligand biologique greffées à l'enrobage.

19. Nanoparticules selon l'une des revendications 1 à 18 **caractérisées en ce que** le ou les ligands biologiques greffés sont dérivés de nucléotides, sucres, vitamines, hormones, biotine, streptavidine, ou toute autre molécule organique présentant un intérêt pour la vectorisation biologique.

20. Nanoparticules selon l'une des revendications 1 à 19 **caractérisées en ce que** des molécules luminescentes ou molécules complexantes, autres que le(s) ligands biologiques, sont greffées à l'enrobage.

21. Nanoparticules selon l'une des revendications 1 à 20 **caractérisées en ce que** des molécules polaires ou chargées du type organophosphonates, amines quaternaires, sont greffées à l'enrobage.

22. Nanoparticules selon l'une des revendications 1 à 20 **caractérisées en ce que** des molécules de polymère solubles dans l'eau de masse moléculaire inférieure à 5000 g/mol sont greffées à l'enrobage.

23. Suspension colloïdale de nanoparticules hybrides selon l'une des revendications 1 à 22.

24. Procédé de préparation d'une suspension colloïdale de nanoparticules hybrides selon la revendication 23, **caractérisé en ce qu'**il comprend les étapes successives suivantes :
a) préparer une suspension colloïdale de nanosphères, de diamètre moyen compris dans la gamme allant de 2 à 9 nm, composées, à au moins 90% en poids de Ln₂O₃, avec Ln qui représente une terre rare, une terre rare dopée avec une terre rare ou un actinide, un mélange de terres rares, ou bien un mélange de terre rare et d'actinide dans lequel au moins 50% des ions métalliques sont des ions terre rare,
b) ajouter à la suspension colloïdale la quantité nécessaire d'un mélange d'organoalcoxysilane et d'agent réticulant pour former à la surface des particules un enrobage, constitué majoritairement de polysiloxane fonctionnalisé avec au moins un groupe réactif, qui présente une épaisseur moyenne comprise dans la gamme allant de 0,5 à 10 nm, et dans lequel de 5 à 75 % des atomes de silicium sont liés à quatre autres atomes de silicium par des ponts oxygène et,
c) greffer chimiquement à l'enrobage au moins un ligand biologique par couplage avec un groupe réactif présent en surface de l'enrobage.

25. Procédé de préparation selon la revendication 24, **caractérisé en ce que** à l'étape a) une suspension colloïdale contenant entre 100 mg et 100 g de nanosphères par litre de solvant est préparée par dissolution de précurseurs de terre rare et/ou d'actinides, dans un solvant polaire et chauffage à une température comprise entre 130 et 250°C, en présence de la quantité d'eau au moins nécessaire pour la formation du sesquioxyde désiré.

26. Procédé selon la revendication 25, **caractérisé en ce que** les précurseurs de terre rare et ou d'actinides sont du type chlorure, acétate ou nitrate.

27. Procédé selon l'une des revendications 24 à 26, **caractérisé en ce que** à l'étape b), le tétraéthyl orthosilicate (TEOS) est utilisé comme agent réticulant.

28. Procédé selon l'une des revendications 24 à 27 **caractérisé en ce qu'**une partie des molécules d'organoalcoxysilane utilisées sont liées de façon covalente à une molécule luminescente.

29. Procédé selon l'une des revendications 24 à 28, **caractérisé en ce que** l'étape c) est précédée d'une étape de greffage de molécules luminescentes et/ou de molécules complexantes et/ou de molécules polaires ou chargées et/ou de molécules de polymères solubles dans l'eau, par couplage avec un groupe réactif présent en surface de l'enrobage.

## Claims

1. Hybrid nanoparticles containing:
- a nanosphere, of mean diameter included in the range from 2 to 9 nm, of which at least 90 % by weight consists of Ln₂O₃, where Ln represents a rare earth, a rare earth doped with a rare earth or an actinide, a mixture of rare earths, or a rare earth and actinide mixture in which at least 50 % of the metal ions are rare earth ions,
- a coating around the nanosphere chiefly consisting of functionalized polysiloxane, having a mean thickness included in the range from 0.5 to 10 nm, and in which from 5 to 75 % of the silicon atoms are bound to four other silicon atoms by oxygen bridges, and
- at least one biological ligand grafted by covalent bonding to the polysiloxane coating.

2. Nanoparticles as in claim 1, **characterized in that** the coating has a density included in the range from 1.6 to 2.4.

3. Nanoparticles as in claim 1, **characterized in that** the coating has a density of less than 2.

4. Nanoparticles as in any of claims 1 to 3, **characterized in that** between 10 and 100 000 luminescent organic molecules are grafted, by covalent bonding, to the coating.

5. Nanoparticles as in claim 4, **characterized in that** the fluorescent organic molecules are chosen from among the derivatives of rhodamine or fluorescein.

6. Nanoparticles as in any of claims I to 5, **characterized in that** the nanosphere, for at least 80 % by weight, consists of a rare earth sesquioxyde, or of a doped rare earth sesquioxide.

7. Nanoparticles as in claim 6, **characterized in that** the nanosphere, four at least 80 % by weight, consists of Gd₂O₃.

8. Nanoparticles as in claim 6, **characterized in that** the nanosphere, for at least 80 % by weight, consists of Y₂O₃.

9. Nanoparticles as in any of claims 1 to 8, **characterized in that** the nanosphere is doped with a lanthanide of type Eu, Tb, Er, Nd, Yb, Tm representing from 0.1 to 25 % of the metal cations.

10. Nanoparticles as in claim 9, **characterized in that** the nanosphere is doped with a lanthanide of type Nd or Yb.

11. Nanoparticles as in claim 9, **characterized in that** the nanosphere is doped with a lanthanide of type Er.

12. Nanoparticles as in any of claims 1 to 8, **characterized in that** the nanosphere is doped with at least two different lanthanides representing from 0.1 to 25 % of the metal cations, at least one of these lanthanides being chosen from among Eu and Tb.

13. Nanoparticles as in any of claims 1 to 6, **characterized in that** more than 10 % of the metal cations of the nanosphere are lanthanide cations having magnetic behaviour, chosen from among Gd, Nd.

14. Nanoparticles as in any of claims 1 to 6, **characterized in that** more than 50 % of the metal cations of the nanosphere are lanthanide cations having a magnetic behaviour chosen from among Gd, Nd.

15. Nanoparticles as in any of claims 1 to 6, **characterized in that** from 0.01 % to 50 % of the metal cations of the nanosphere are actimide cations chosen from among Ac, Th, Pa, U, Np, Pu.

16. Nanoparticles as in any of claims I to 15, **characterized in that** at least 1 % of the metal cations of the nanopshere having extensive neutron-capture capability, chosen for exemple from among the isotopes ¹⁵⁷Gd and ²³⁵U.

17. Nanoparticles as in any of claims 1 to 16, **characterized in that** from 1 to 1000 molecules of biological ligand are grafted onto the coated by covalent bonding.

18. Nanoparticles as in any of claims 1 to 16, **characterized in that** less than 10 % by weight of these nanoparticles contain more than two molecules of biological ligand grafted onto the coating.

19. Nanoparticles as in any of claims 1 to 18, **characterized in that** the grafted biological ligand or ligands are derived from nucleotides, sugars, vitamins, hormones, biotin, streptavidin, or any other organic molecule of interest for biological vectoring.

20. Nanoparticles as in any of claims 1 to 19, **characterized in that** luminescent molecules or complexing molecules other than the biological ligand(s) are grafted onto the coating.

21. Nanoparticles as in any of claims 1 to 20, **characterized in that** polar or charged molecules of organophosphate, quaternary amine types are grafted onto the coating.

22. Nanoparticles as in any of claims 1 to 20, **characterized in that** molecules of water-soluble polymers having a molecular weight of less than 5000 g/mol are grafted onto the coating.

23. Colloidal suspension of hybrid nanoparticles as in any of claims 1 to 22.

24. Method for preparing a colloidal suspension of hybrid nanoparticules as in claim 23, **characterized in that** it comprises the following successive steps:
a) preparing a colloidal suspension of nanospheres, of mean diameter included in the range from 2 to 9 nm consisting, for at least 90 wt.%, of Ln₂O₃, where Ln represents a rare earth, a rare earth doped with a rare earth or an actinide, a mixture of rare earths, or a rare earth and actinide mixture in which at least 50 % of the metal ions are rare earth ions,
b) adding to the colloidal suspension the necessary quantity of a mixture of organoalcoxysilane and cross-linking agent to form a coating on the surface of the particles, chiefly consisting of polysiloxane functionalized with at least one reactive group, having a mean thickness included in the range from 0.5 to 10 nm, and in which from 5 to 75 % of the silicon atoms are bound to four other silicon atoms by oxygen bridges, and
c) chemically grafting at least one biological ligand to the coating, by coupling with a reactive group present on the coating surface.

25. Preparation method as in claim 24, **characterized in that** at step a) a colloidal suspension containing between 100 mg and 100 g of nanospheres per litre of solvent is prepared by dissolving precursors of rare earth and/or actinides in a polar solvent, and heating to a temperature of between 130 and 250°C, in the presence of the quantity of water at least necessary to form the desired sesquioxide.

26. Method as in claim 25, **characterized in that** the precursors of rare earth or actinides are of chloride, acetate or nitrate type.

27. Method as in any of claims 24 to 26, **characterized in that** at step b), orthosilicate tetraethyl (TEOS) is used as cross-linking agent.

28. Method as in any of claims 24 to 27, **characterized in that** part of the molecules of organoalcoxysilane used are covalently bound to a luminescent molecule.

29. Method as in any of claims 24 to 28, **characterized in that** step c) is preceded by a grafting step of luminescent molecules and/or complexing molecules and/or polar or charged molecules and/or molecules of water-soluble polymers, by coupling with a reactive group present on the coating surface.

## Patentansprüche

1. Hybridnanopartikel, umfassend:
- eine Nanosphäre mit einem mittleren Durchmesser im Bereich zwischen 2 und 9 nm, die zu wenigstens 90 Gew.-% aus Ln₂O₃ besteht, mit Ln, das eine Seltene Erde, eine mit einer Seltenen Erde oder einem Actinoid dotierte Seltene Erde, eine Mischung aus Seltenen Erden oder aber eine Mischung aus Seltener Erde und Actinoid, worin wenigstens 50 % der Metallionen Seltenerdionen sind, darstellt,
- eine Ummantelung um die Nanosphäre, die hauptsächlich aus funktionalisiertem Polysiloxan besteht und die eine mittlere Dicke im Bereich zwischen 0,5 und 10 nm aufweist und worin 5 bis 75 % der Siliziumatome durch Sauerstoffbrücken an vier weitere Siliziumatome gebunden sind, und
- wenigstens einen biologischen Liganden, der durch kovalente Bindung auf die Polysiloxan-Ummantelung gepfropft ist.

2. Nanopartikel nach Anspruch 1, **dadurch gekennzeichnet, daß** die Ummantelung eine Dichte im Bereich zwischen 1,6 und 2,4 aufweist.

3. Nanopartikel nach Anspruch 1, **dadurch gekennzeichnet, daß** die Ummantelung eine Dichte von weniger als 2 aufweist.

4. Nanopartikel nach einem der Ansprüche 1 bis 3, **dadurch gekennzeichnet, daß** zwischen 10 und 100.000 lumineszierende organische Moleküle durch kovalente Bindung auf die Ummantelung gepfropft sind.

5. Nanopartikel nach Anspruch 4, **dadurch gekennzeichnet, daß** die fluoreszierenden organischen Moleküle aus den Derivaten des Rhodamin oder des Fluorescein ausgewählt sind.

6. Nanopartikel nach einem der Ansprüche 1 bis 5, **dadurch gekennzeichnet, daß** die Nanosphäre zu wenigstens 80 Gew.-% aus Sesquioxid von Seltener Erde oder aus Sesquioxid von dotierter Seltener Erde besteht.

7. Nanopartikel nach Anspruch 6, **dadurch gekennzeichnet, daß** die Nanosphäre zu wenigstens 80 Gew.-% aus Gd₂O₃ besteht.

8. Nanopartikel nach Anspruch 6, **dadurch gekennzeichnet, daß** die Nanosphäre zu wenigstens 80 Gew.-% aus Y₂O₃ besteht.

9. Nanopartikel nach einem der Ansprüche 1 bis 8, **dadurch gekennzeichnet, daß** die Nanosphäre mit einem Lanthanoid vom Typ Eu, Tb, Er, Nd, Yb, Tm, das 0,1 bis 25 % der Metallkationen ausmacht, dotiert ist.

10. Nanopartikel nach Anspruch 9, **dadurch gekennzeichnet, daß** die Nanosphäre mit einem Lanthanoid vom Typ Nd oder Yb dotiert ist.

11. Nanopartikel nach Anspruch 9, **dadurch gekennzeichnet, daß** die Nanosphäre mit einem Lanthanoid vom Typ Er dotiert ist.

12. Nanopartikel nach einem der Ansprüche 1 bis 8, **dadurch gekennzeichnet, daß** die Nanosphäre mit wenigstens zwei unterschiedlichen Lanthanoiden dotiert ist, die 0,1 bis 25 % der Metallkationen ausmachen, wobei wenigstens eines dieser Lanthanoide aus Eu und Tb ausgewählt ist.

13. Nanopartikel nach einem der Ansprüche 1 bis 6, **dadurch gekennzeichnet, daß** mehr als 10 % der Metallkationen der Nanosphäre Kationen von Lanthanoid mit einem magnetischen Verhalten, ausgewählt aus Gd, Nd sind.

14. Nanopartikel nach einem der Ansprüche 1 bis 6, **dadurch gekennzeichnet, daß** mehr als 50 % der Metallkationen der Nanosphäre Kationen von Lanthanoid mit einem magnetischen Verhaften, ausgewählt aus Gd, Nd sind.

15. Nanopartikel nach einem der Ansprüche 1 bis 6, **dadurch gekennzeichnet, daß** 0,01 % bis 50 % der Metallkationen der Nanosphäre Actinoid-Kationen, ausgewählt aus Ac, Th, Pa, U, Np, Pu sind.

16. Nanopartikel nach einem der Ansprüche 1 bis 15, **dadurch gekennzeichnet, daß** wenigstens 1 % der Metallkationen der Nanosphäre eine hohe Neutroneneinfangfähigkeit aufweisen, die aus den Isotopen ¹⁵⁷Gd und ²³⁵U ausgewählt sind.

17. Nanopartikel nach einem der Ansprüche 1 bis 16, **dadurch gekennzeichnet, daß** 1 bis 1000 Bioliganden-Moleküle durch kovalente Bindung auf die Ummantelung gepfropft sind.

18. Nanopartikel nach einem der Ansprüche 1 bis 16, **dadurch gekennzeichnet, daß** weniger als 10 Gew.-% dieser Nanopartikel mehr als zwei auf die Ummantelung gepfropft Bioliganden-Moleküle umfassen.

19. Nanopartikel nach einem der Ansprüche 1 bis 18, **dadurch gekennzeichnet, daß** der oder die aufgepfropften biologischen Liganden von Nukleotiden, Zuckern, Vitaminen, Hormonen, Biotin, Streptavidin oder jedem anderen organischen Molekül, das für die biologische Vektorisierung von Interesse ist, abgleitet sind.

20. Nanopartikel nach einem der Ansprüche 1 bis 19, **dadurch gekennzeichnet, daß** lumineszierende Moleküle oder komplexbildende Moleküle, andere als der (die) bio!ogische(n) Ligand(en), auf die Ummantelung gepfropft sind.

21. Nanopartikel nach einem der Ansprüche 1 bis 20, **dadurch gekennzeichnet, daß** polare oder beladene Moleküle vom Typ Organophosphonate, quaternäre Amine auf die Ummantelung gepfropft sind.

22. Nanopartikel nach einem der Ansprüche 1 bis 20, **dadurch gekennzeichnet, daß** wasserlösliche Polymermoleküle mit einer molaren Masse von weniger als 5000 g/mol auf die Ummantelung gepfropft sind.

23. Kolloidale Suspension von Hybridnanopartikeln nach einem der Ansprüche 1 bis 22.

24. Verfahren zur Herstellung einer kolloidalen Suspension von Hybridnanopartikeln nach Anspruch 23, **dadurch gekennzeichnet, daß** es die folgenden aufeinanderfolgenden Schritte umfaßt:
a) Zubereiten einer kolloidalen Suspension von Nanosphären mit einem mittleren Durchmesser im Bereich zwischen 2 und 9 nm, die zu wenigstens 90 Gew.-% aus Ln₂O₃ bestehen, mit Ln, das eine Seltene Erde, eine mit einer Seltenen Erde oder einem Actinoid dotierte Seltene Erde, eine Mischung aus Seltenen Erden oder aber eine Mischung aus Seltener Erde und Actinoid, worin wenigstens 50 % der Metallionen Seltenerdionen sind, darstellt,
b) Zugeben -zu der kolloidalen Suspension- der erforderlichen Menge einer Mischung aus Organoalkoxysilan und Vernetzungsmittel, um auf der Oberfläche der Partikel eine Ummantelung, hauptsächlich bestehend aus mit wenigstens einer reaktiven Gruppe funktionalisiertem Polysiloxan, zu bilden, die eine mittlere Dicke im Bereich zwischen 0,5 und 10 nm aufweist und worin 5 bis 75 % der Siliziumatome durch Sauerstoffbrücken an vier weitere Siliziumatome gebunden sind, und
c) chemisches Aufpfropfen -auf die Ummantelung- wenigstens eines biologischen Liganden durch Kopplung mit einer reaktiven Gruppe, die an der Oberfläche der Ummantelung vorliegt.

25. Herstellungsverfahren nach Anspruch 24, **dadurch gekennzeichnet, daß** bei Schritt a) eine kolloidale Suspension, die zwischen 100 mg und 100 g Nanosphären pro Liter Lösungsmittel enthält, durch Auflösen von Präkursoren von Seltener Erde und/oder von Actinoiden in einem polaren Lösungsmittel und Erhitzen auf eine Temperatur zwischen 130 und 250 °C, in Anwesenheit der wenigstens für die Bildung des gewünschten Sesquioxids erforderlichen Wassermenge, hergestellt wird.

26. Verfahren nach Anspruch 25, **dadurch gekennzeichnet, daß** die Präkursoren von Seltener Erde und/oder von Actinoiden vom Typ Chlorid, Acetat oder Nitrat sind.

27. Verfahren nach einem der Ansprüche 24 bis 26, **dadurch gekennzeichnet, daß** bei Schritt b) Tetraethylorthosilikat (TEOS) als Vernetzungsmittel verwendet wird.

28. Verfahren nach einem der Ansprüche 24 bis 27, **dadurch gekennzeichnet, daß** ein Teil der verwendeten Organoalkoxysilan-Moleküle kovalent an ein lumineszierendes Molekül gebunden sind.

29. Verfahren nach einem der Ansprüche 24 bis 28, **dadurch gekennzeichnet, daß** dem Schritt c) ein Schritt zum Aufpfropfen von lumineszierenden Molekülen und/oder von komplexbildenden Molekülen und/oder von polaren oder beladenen Molekülen und/oder von wasserlöslichen Polymermolekülen, durch Kopplung mit einer an der Oberfläche der Ummantelung vorliegenden reaktiven Gruppe vorausgeht.
